# EUROPEAN PATENT SPECIFICATION

(11) **EP 3 391 839 B1**
(45) Date of publication and mention of the grant of the patent: **17.02.2021**
(21) Application number: 16874655.0
(22) Date of filing: 31.10.2016
(51) Int. Cl.: A61B 17/32, A61B 90/00

(54) **ADJUSTABLE AND BENDABLE TISSUE REMOVAL APPARATUS**
EINSTELLBARE UND BIEGBARE GEWEBEENTNAHMEVORRICHTUNG
APPAREIL D'ABLATION TISSULAIRE RÉGLABLE ET SOUPLE

(30) Priority: 15.12.2015 CN 201510929256
(43) Date of publication of application: 24.10.2018
(73) Proprietor: Ningbo Hicren Biotechnology Co. Ltd., Ningbo, Zhejiang 315336 (CN)
(72) Inventor: LV, Shiwen, Ningbo Zhejiang 315336 (CN); MAO, Keya, Ningbo Zhejiang 315336 (CN); DENG, Shuang, Ningbo Zhejiang 315336 (CN); WANG, Yu, Ningbo Zhejiang 315336 (CN)
(74) Representative: Sadler, Peter Frederick
(86) International application number: PCT/CN2016/103989
(87) International publication number: WO 2017/101598

(56) References cited:
- WO-A1-2014/041540
- WO-A2-2015/168176
- CN-A- 103 702 626
- CN-A- 104 473 687
- CN-A- 105 125 241
- CN-A- 105 125 241
- CN-A- 105 534 573
- CN-A- 105 662 540
- US-A- 5 282 821
- US-A1- 2003 225 364
- US-A1- 2010 298 832
- US-A1- 2013 110 112

## Description

### RELATED APPLICATION

This present application claims the benefit of priority of Chinese Patent Application No. 201510929256.5, filed on December 15, 2015 and entitled "Adjustable and Bendable Tissue Removal Apparatus".

### TECHNICAL FIELD

The present invention relates to the technical field of spinal stenosis decompression apparatus, and more particularly, to a new medical adjustable and bendable tissue removal apparatus.

### BACKGROUND

Human tissues usually undergo hyperplasia and degeneration with age, which will result in spinal stenosis or nerve root canal stenosis, causing pressure on the cauda equina or on the nerve root, thereby causing numbness, pains, and even severe paralysis of the patient. In a spine surgery, the patient's hyperplastic or degenerated tissues need to be polished or cut, so as to reduce the pressure on the nerve exerted by the tissue, thereby alleviating and eliminating the effects caused by the pressure.

In the prior art, for example, as disclosed by the International Patent Publication WO 2014/041540 A1, the powered handle drives the cutting head to rotate , grind and cut, and drive shaft sleeve sleeved outside the drive shaft has a non-adjustable and fixed bending shape.

In a spine surgery, it is often required to grind and cut on multiple surgical sites, and all of these sites are rather deep, so it is difficult for a fixed-shaped working sheath to go deep to arrive at the multiple specified sites through a minimally invasive surgical channel; especially, the outer shape of the portion to be ground is complex, and it is difficult to control the cutting head to adapt to the outer shape of the portion to be ground. In addition, the device is equipped with a shield, but the shield is fixed relative to the device. As different surgeries have different requirements for the direction and configuration of the shield, it is difficult for the device to adapt to different surgical situations.

In the prior art, for example, as disclosed by the Chinese Patent Publication CN 103767759 A, a bendable grinding apparatus controls the grinding head to bend and lift, so as to adapt to the change of the site of the portion to be ground.

The end of the working sheath of the apparatus is flat, and no protective devices are provided in operation, so the apparatus is apt to damage the surrounding tissues or nerves when cutting the affected parts. In addition, the diameter of the distal end of the apparatus is larger, but in a minimally invasive surgery, it is required that the outer diameter of the distal end of the apparatus is smaller, so as to adapt to the tiny working channel and reduce surgical risks.

### SUMMARY

The invention is defined by independent claim 1 and preferred embodiments are listed in the dependent claims.

In view of this, the objective of the present invention is to provide a medical adjustable and bendable tissue removal apparatus, which can adjust the angle of the tissue removal head during surgery and has good adaptability. What's more, a protective cover is arranged on the side of the tissue removal head. The position of the protection cover relative to the tissue removal head and the axis of the tissue removal head can be adjusted through the protection control member and the control handle, so as to prevent the apparatus from cutting away the tissues unnecessary to be removed. Additionally, the outer wall of the distal end of the protection control member and the adjustable and bendable unit are set eccentrically, so as to reduce the size of the distal end and reduce the surgical risks.

The technical schemes are as follows:
An adjustable and bendable tissue removal apparatus, comprising a tissue removal head, an adjustable and bendable unit, a rotation shaft, and a straight adjusting pipe, the adjustable and bendable unit includes a straight segment at a proximal end and a fixed bending part at a distal end; a proximal end of the straight segment is fixedly connected with a regulation handle; the fixed bending part is made of elastic material; the rotation shaft is arranged inside the adjustable and bendable unit; a distal end of the rotation shaft is connected with the tissue removal head, and a proximal end of the rotation shaft is provided with a connection member that is connected with an output shaft of the motor; the straight adjusting pipe is sleeved outside the adjustable and bendable unit; a proximal end of the straight adjusting pipe is connected with the regulation handle; the straight adjusting pipe can be controlled to move axially by operating the regulation handle ; the adjustable and bendable tissue removal apparatus further includes a protective device; the protective device comprises a protective cover, a protection control member and a control handle; the protective cover is arc sheet-shaped; a proximal end of the protection cover is connected with a distal end of the protection control member, and the distal end of the protection control member is flexible and has a ring-shaped structure; a proximal end of the protection control member is connected with the control handle; the protection cover is controlled to move axially or rotate by operating the control handle; a distance between an inner wall of the protective cover and an axis of the tissue removal head is greater than a radius of the tissue removal head.

Preferably, the ring-shaped structure is an open ring-shaped structure, and a width of an opening of the open ring-shaped structure is smaller than an outer diameter of the adjustable and bendable unit.

Preferably, a distance from an outer side wall of the ring-shaped structure, which is located at a same side where the protective cover is disposed, to an axis of the adjustable and bendable unit, is greater than a distance from an opposite outer side wall of the ring-shaped structure to the axis of the adjustable and bendable unit.

Preferably, a circumferential wall thickness of the ring-shaped structure is nonuniform, and the protection cover is fixed at a side where the wall is thicker.

Preferably, protrusions are provided on an inner wall of the ring-shaped structure, and the protrusion is dot-shaped or strip-shaped; the protective cover is disposed on a same side where the protrusions are disposed.

Preferably, the protection device further includes a gasket which is disposed between the protection control member and the adjustable and bendable unit; the protective cover is fixed on a same side where the gasket is arranged.

Preferably, the protection control member is disposed between the straight adjusting pipe and the adjustable and bendable unit, or arranged outside the adjusting straight pipe.

Preferably, a support component is provided at a distal end of the adjustable and bendable unit; the support component has a mounting hole for mounting the tissue removal head, and the axis of the mounting hole coincides with the axis of the distal end of the fixed bending part.

Preferably, a longitudinal section of an intermediate portion of the fixed bending part is arc sheet-shaped in structure.

Preferably, a sheet-shaped structure is disposed on an inner side in the bending direction of the adjustable and bendable unit.

Preferably, a sheet-shaped structure is disposed on an outer side in the bending direction of the adjustable and bendable unit; the adjustable and bendable unit is sleeved with a protective sleeve, which is bendable along with the bending of the fixed bending part.

Preferably, the tissue removal head is a grinding head, and a distal end of the rotation shaft is configured to be bended along with bending of the fixed bending part.

Preferably, a distal end of the rotation shaft is formed by winding multiple layers of filaments tightly; each layer is formed by winding a plurality of filaments spirally, and winding directions of adjacent filament layers are opposite.

Preferably, a liquid absorbing channel or inpouring channel is disposed between the rotation shaft and the adjustable and bendable unit.

Preferably, a water tank is arranged inside the handle, and the water tank communicates with the proximal end of the adjustable and bendable unit.

In some of the embodiments, the adjustable and bendable tissue removal apparatus further includes a position limiting device; the position limiting device is provided on the protection control member or on the control handle; when the protective cover moves towards the tissue removal head, the position limiting device is configured to prevent the protective cover from completely passing across the tissue removal head.

The present invention has the following beneficial effects over the prior art:
1. The present invention realizes the controllable change of the direction of the tissue removal head, which enables the tissue removal head to bend according to demands, thereby expanding the range of tissue removal by the apparatus during a minimally invasive surgery, and making the operation more flexible and convenient.
2. In the present invention, a protective cover is arranged at a side of the tissue removal head, and the position of the protective cover relative to the tissue removal head and the axis thereof can be adjusted by the protection control member and the control handle, thereby effectively preventing the apparatus from removing tissues unnecessary to be removed, such as nerves, etc.; in the process of removing the tissues, the cooling water may be guided to the site of the portion to be cut by the protective cover, thereby lowering the temperature of the portion to be cut through flushing.
3. In the present invention, the outer wall of the distal end of the protection control member and the adjustable and bendable unit are set eccentrically, thereby reducing the size of the distal end of the apparatus, making the operation of the apparatus in a tiny surgical channel more flexible, and reducing surgical risks.

### BRIEF DESCRIPTION OF THE DRAWINGS

In order to make the content of the present invention more clear and better understood, the present disclosure will be further described in more details with reference to the accompanying figures and embodiments, wherein,
FIG. 1 is a schematic structural view of an adjustable and bendable tissue removal apparatus according to the present disclosure;
FIG. 2A is a schematic structural view illustrating a straight and arc sheet-shaped protective cover according to the present disclosure;
FIG. 2B is a schematic structural view illustrating a bent and arc sheet-shaped protective cover according to the present disclosure;
FIG. 3A is a schematic structural view illustrating a closed ring-shaped structure according to the present disclosure;
FIG. 3B is a schematic structural view illustrating an open ring-shaped structure according to the present disclosure;
FIG. 4A is a schematic view illustrating a tubular structure between the distal end of the protection control member and the control handle according to the present disclosure;
FIG. 4B is a schematic view illustrating a sheet-shaped structure between the distal end of the protection control member and the control handle according to the present disclosure;
FIG. 4C is a schematic view illustrating a sheet-shaped structure with a ring-shaped member, which is disposed between the distal end of the protection control member and the control handle according to the present disclosure;
FIG. 5A is a schematic longitudinal sectional view illustrating the ring-shaped structure with nonuniform circumferential wall thickness according to the present disclosure;
FIG. 5B is a schematic cross sectional view illustrating the ring-shaped structure with nonuniform circumferential wall thickness according to the present disclosure;
FIG. 6A is a schematic longitudinal sectional view illustrating the inner wall of the ring-shaped structure with protrusions according to the present disclosure;
FIG. 6B is a schematic cross sectional view illustrating the inner wall of the ring-shaped structure with protrusions according to the present disclosure;
FIG. 7A is a schematic longitudinal sectional view illustrating the protection control member provided with a gasket according to the present disclosure;
FIG. 7B is a schematic cross sectional view illustrating the protection control member provided with a gasket according to the present disclosure;
FIG. 8 is a schematic sectional view of a portion of the regulation handle and the control handle according to the present disclosure.

In the drawings: 1 - tissue removal head; 11-adjustable and bendable unit; 12-rotation shaft; 13-fixed bending part; 15-liquid inpouring channel; 17-support component; 18-straight segment; 20- water tank; 21-motor; 22-protective cover; 23- protection control member; 24- protective sleeve; 25- gasket; 26-water pipe; 31-straight adjusting pipe; 33- screw bolt; 34- threaded sleeve; 35-regulation handle; 36- control handle; 37 position limiting device.

### DETAILED DESCRIPTION OF THE PREFERRED EMBODIMENTS

To make the objectives, technical schemes, and advantages of the present invention clearer, the present disclosure will be further described in more details with reference to the accompanying figures and embodiments.

The proximal end in the present disclosure refers to the end near the surgeon, and the distal end refers to the end far away from the surgeon.

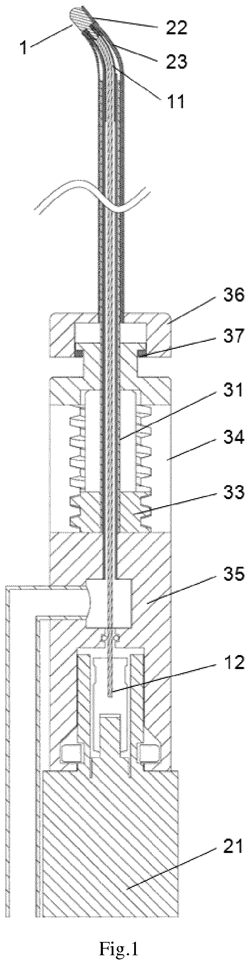

As shown in FIG. 1 and FIGS. 5 to 7, a medical adjustable and bendable tissue removal apparatus includes a tissue removal head 1, an adjustable and bendable unit 11, a rotation shaft 12, and a straight adjusting pipe 31. The adjustable and bendable unit 11 includes a straight segment 18 at the proximal end and the fixed bending part 13 at the distal end. A proximal end of the straight segment 18 is fixedly connected with the regulation handle 35. The fixed bending part 13 is made of elastic material. The rotation shaft 12 is arranged inside the adjustable and bendable unit 11. The distal end of the rotation shaft is connected with the tissue removal head 1, and the proximal end of the rotation shaft 12 is provided with a connection member that is connected with the output shaft of the motor. The straight adjusting pipe 31 is sleeved outside the adjustable and bendable unit 11.

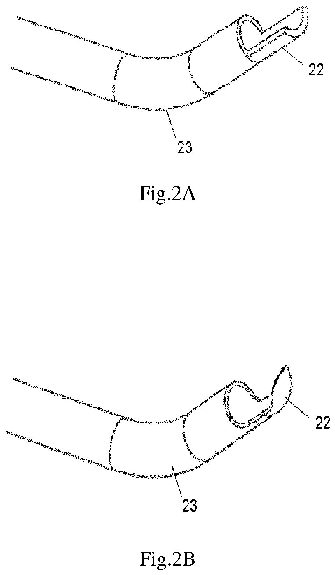

As shown in FIGS. 2A and 2B, the present invention further includes a protective device. The protective device comprises an arc sheet-shaped protective cover 22. The distance between the inner wall of the protective cover 22 and the axis of the tissue removal head 1 is greater than the radius of the tissue removal head 1, so as to avoid mutual interference between the tissue removal head 1 and the protective cover 22. The circumferential width protected by the protective cover 22 is less than or equal to half of the circumference of the tissue removal head 1, so as to avoid damages to tissues such as nerves unnecessary to be removed. Wherein, as shown in FIG. 2A, the protective cover 22 is a straight arc-shaped sheet, which can protect the tissues at the side where the tissue removal head 1 is located. As shown in FIG. 2B, the protective cover 22 is bent arc sheet-shaped, and it can not only protect the tissues at the side, but also the tissues at the front end.

In different surgeries, the protection direction of the protective cover and the necessity for providing the protective cover are different. The protective device further includes a protection control member 23 and a control handle 36, which are configured to control and adjust the protective cover. As shown in FIG. 1, the proximal end of the protection cover 22 is connected with the distal end of the protection control member 23, and the distal end of the protection control member 23 is flexible and has a ring-shaped structure. The proximal end of the protection control member 23 is connected with the control handle 36. Operate the control handle 36 to make the distal end of the protection control member 23 move axially or rotate around the distal end of the adjustable and bendable unit 11, thereby driving the protective cover 22 to move axially or rotate.

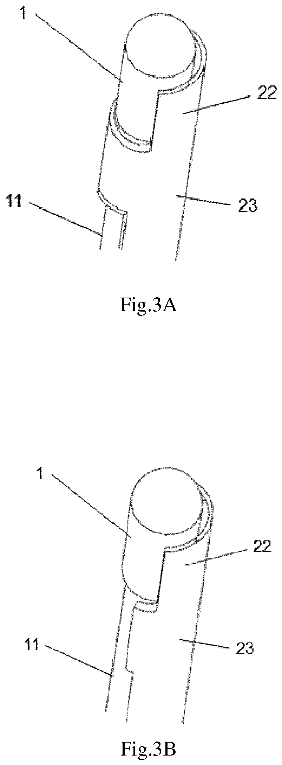

In order to enable the protection control member 23 to move axially or rotate around the distal end of the adjustable and bendable unit 11, the distal end of the protection control member 23 is flexible and has a ring-shaped structure. As shown in FIG. 3A. the ring-shaped structure is closed and rounds the distal end of the adjustable and bendable unit. As shown in FIG. 3B, the ring-shaped structure is an open ring-shaped structure, and the width of the opening of the open ring-shaped structure is smaller than the outer diameter of the adjustable and bendable unit, so as to stop the distal end of the protection control member from breaking away from the adjustable and bendable unit during rotation, causing the apparatus not to be used normally.

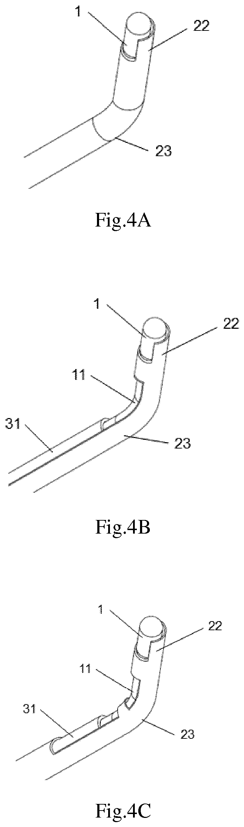

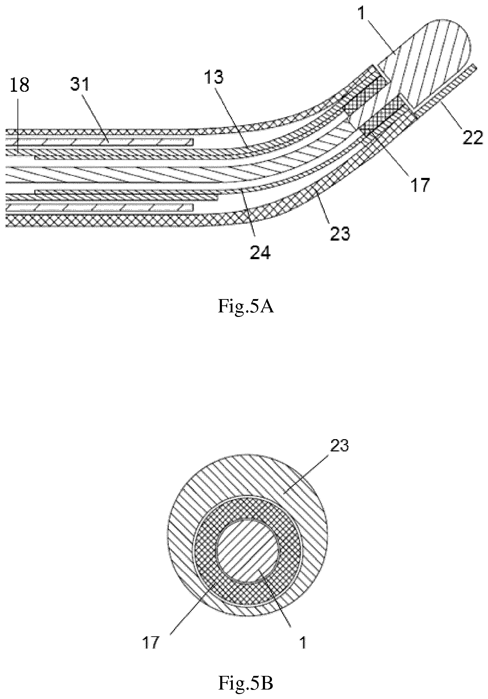

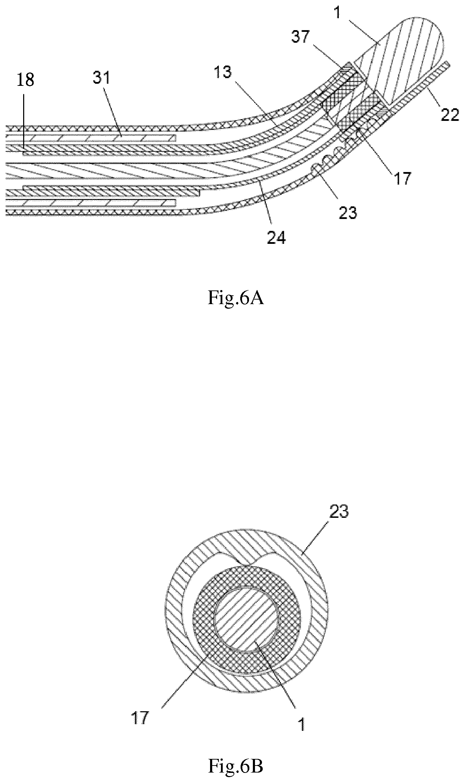

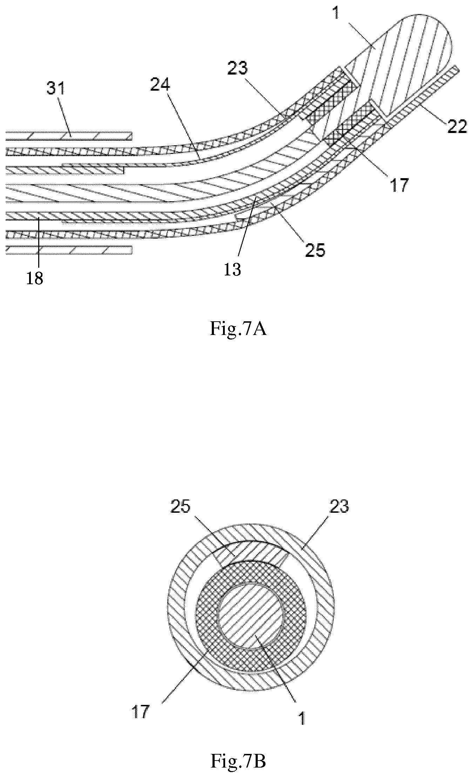

As shown in FIG. 4A, the connecting segment of the proximal end of the protection control member 23 and the control handle 36 may be tubular. In order to save material, as shown in FIG. 4B, the connecting segment of the proximal end of the protection control member 23 and the control handle 36 may be sheet-shaped. As shown in FIG. 4C, the connecting segment of the proximal end of the protection control member 23 and the control handle 36 may be a sheet-shaped and provided with a ring-shaped member. As shown in FIG. 5A and FIG. 6A, the protection control member 23 may be arranged outside the straight adjusting pipe 31. As shown in FIG. 7A, the protection control member 23 may also be arranged between the straight adjusting pipe 31 and the adjustable and bendable unit 11.

In order to reduce the size of the cross section of the distal end and reduce the surgical risks, the distance from the outer side wall of the ring-shaped structure at the distal end of the protection control member 23, which is disposed at the same side where the protective cover is disposed, to the axis of the adjustable and bendable unit, is greater than the distance from the opposite outer side wall of the ring-shaped structure to the axis of the bending unit. The specific embodiment is as follows:
As shown in FIG. 5A and FIG. 5B, the protection control member 23 is arranged outside the straight adjusting pipe 31, and the inner diameter of the ring-shaped structure disposed at the distal end matches with the outer diameter of the distal end of the fixed bending portion 13. The circumferential wall thickness of the ring-shaped structure is nonuniform, and the protection cover 22 is fixed at the side where the wall is thicker. The proximal end of the protection control member 23 is connected with the control handle 36. Operate the control handle 36 to enable the protection control member 23 to rotate or move axially, thereby driving the protective cover 22 to move.

As shown in FIG. 6A and FIG. 6B, protrusions are provided on the inner wall of the ring-shaped structure at the distal end of the protection control member 23, and the protrusion is dot-shaped, or may also be strip-shaped. The protective cover 22 is disposed on the same side where the protrusions are disposed.

As shown in FIG. 7A and FIG. 7B, the protection control member 23 is disposed between the straight adjusting pipe 31 and the adjustable and bendable unit 11, and the protection device further includes a gasket 25 which is disposed between the protection control member 23 and the adjustable and bendable unit 11. The protective cover 22 is fixed on the same side where the gasket 25 is arranged.

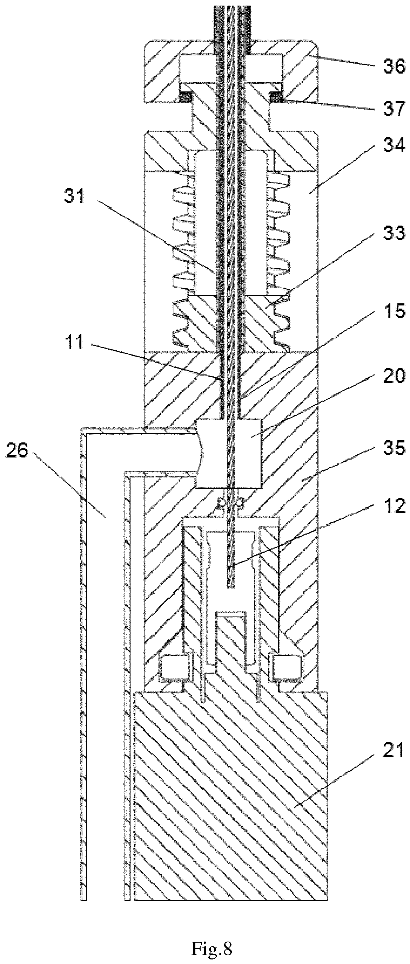

In order to prevent the protective cover 22 from completely passing across the tissue removal head 1, and prevent the protection control member 23 from interfering the operation of the tissue removal head 1, the medical adjustable and bendable tissue removal device further includes a position limiting device 37. The position limiting device 37 may be provided on the protection control member 23 or the control handle 36. As shown in FIG. 8, the position limiting device 37 is arranged on the inner side of the control handle 36. Cooperating with the regulation handle 35, the position limiting device 37 prevents the control handle 36 from moving axially, thereby stopping the protective cover 22 from excessively stretching forwardly.

As shown in FIG. 6A, the position limiting device 37 is arranged at the distal end of the protection control member 23, and is fixedly connected with the distal end of the fixed bending part 13. The outer diameter of the position limiting device 37 is greater than the inner diameter of the protection control member 23, thereby preventing the protection control member 23 from moving axially and stopping the protective cover 22 from excessively stretching forwardly.

The adjustable and bendable unit 11 can be implemented in various ways.

As shown in FIG. 5A and FIG. 6A, a longitudinal section of the intermediate portion of the fixed bending part 13 is arc sheet-shaped in structure. The arc sheet-shaped structure is arranged at the inner side of the adjustable and bendable unit 11. In the process of moving human tissue, in order to prevent the rotation shaft 12 from emerging from the gap of the fixed bending part 13 to affect the operation of the apparatus, a protective sleeve 24 is provided. The outer diameter of the rotating shaft 12 matches with the inner diameter of the fixed bending part 13, so as to close the fixed bending part 13.

As shown in FIG. 7A, the sheet-shaped structure is disposed on the outer side in the bending direction of the adjustable and bendable unit 11. The adjustable and bendable unit 11 is sleeved with a protective sleeve 24, whose inner diameter matches with the outer diameter of the fixed bending part 13, so as to close the fixed bending part 13. The protective sleeve 24 can bend in synchronization with the fixed bending part 13.

The distal end of the adjustable and bendable unit 11 is provided with a tissue removal head 1. As shown in FIG. 5A, in order to make the tissue removal head be more securely mounted, a support component 17 is provided at the distal end of the adjustable and bendable unit 11. The support component 17 has a mounting hole for mounting the tissue removal head 1, and the axis of the mounting hole coincides with the axis of the distal end of the fixed bending part 13. In order to obtain a better grinding effect, the diameter of the tissue removal head 1 is larger than the outer diameter of the distal end of the fixed bending part 13, so that the cutting edge of the tissue removal head 1 protrudes from the fixed bending part 13.

As shown in FIG. 5A, the tissue removal head 1 is connected with the distal end of the rotation shaft 12. The exemplary method of removing tissue can be grinding, and the tissue removal head 1 is a grinding head accordingly, and the rotation shaft 12 has a flexible distal end, which can bend along with the bending of the fixed bending part 13. The distal end of the rotation shaft 12 is formed by winding multiple layers of filaments tightly. Each layer is formed by winding a plurality of filaments spirally, and winding directions of adjacent filament layers are opposite.

In order to cool the tissue and prevent overheat of the ground position, a liquid inpouring channel 15 is provided in the tissue removal apparatus. As shown in FIG. 8, there is a gap between the adjustable and bendable unit 11 and the rotation shaft 12, and the gap forms the liquid inpouring channel 15. The cooling water enters the water tank 20 through the water pipe 26, then enters the liquid inpouring channel 15, finally, guided by the protective cover 22, the cooling water arrives at the ground position of the tissue removal head 1 to cool the tissue.

The liquid inpouring channel 15 may also be disposed inside the rotation shaft 12, and the rotation shaft 12 is hollow.

The straight adjusting pipe 31 is sleeved outside the rotation shaft 12 and the adjustable and bendable unit 11, and the proximal end of the straight adjusting pipe 31 is connected with the regulation handle 35. In order to regulate the straight adjusting pipe 31 better, a stretch-contract mechanism is usually arranged on the regulation handle 35. As shown in FIG. 8, the stretch-contract mechanism disposed on the regulation handle 35 includes a screw bolt 33 and a threaded sleeve 34 which are matching. The threaded sleeve 34 is arranged on the regulation handle 35 and connected rotatable with the regulation handle 35. The screw bolt 33 is connected movable with the regulation handle 35. The intermediate portion of the screw bolt 33 is provided with a mounting hole for mounting the straight adjusting pipe 31. The proximal end of the straight adjusting pipe 31 is fixedly connected with the screw bolt 33, and the screw bolt 33 matches with the threaded sleeve 34. When rotate the threaded sleeve 34, the screw bolt 33 slides axially in the guide rail, thereby driving the straight adjusting pipe 31 to move axially.

As shown in FIG. 8, the proximal end of the rotation shaft 12 is provided with a connection member connected with the output shaft of the motor, so that the rotation shaft 12 is connected with the motor 21. The connection member may be a standard connection member specified in the national standard YY-1012.

In practical applications, by means of the control handle 36, the operator can adjust the orientation of the protective cover 22 or the position of the protective cover 22 relative to the axis of the tissue removal head 1, to protect the tissues unnecessary to be cut and to adapt to a complicated surgical environment; by means of the stretch-contract mechanism, the operator can also control the movement of the straight adjusting pipe 31 relative to the adjustable and bendable unit 11, and regulate the distance that the fixed bending part 13 extends from the straight adjusting pipe 31, to change the angle between the tissue removal head 1 disposed at the distal end of the adjustable and adjustable and bendable unit 11 and the straight segment 18, so that the tissue removal head 1 can be controlled to rotate during surgery.

## Claims

1. An adjustable and bendable tissue removal apparatus, comprising a tissue removal head (1), an adjustable and bendable unit (11), a rotation shaft (12), the adjustable and bendable unit (11) includes a straight segment (18) at a proximal end and a fixed bending part (13) at a distal end; a proximal end of the straight segment (18) is fixedly connected with a regulation handle (35); the rotation shaft (12) is arranged inside the adjustable and bendable unit (11); a distal end of the rotation shaft is connected with the tissue removal head (1), and a proximal end of the rotation shaft is provided with a connection member that is connected with an output shaft of the motor; the adjustable and bendable tissue removal apparatus further includes a protective device; wherein the adjustable and bendable tissue removal apparatus further comprises a straight adjusting pipe (31), wherein the straight adjusting pipe (31) is sleeved outside the adjustable and bendable unit (11); a proximal end of the straight adjusting pipe (31) is connected with the regulation handle (35); the straight adjusting pipe (31) can be controlled to move axially by operating the regulation handle (35); and that the fixed bending part (13) is made of elastic material; wherein the protective device comprises a protective cover (22), a protection control member (23) and a control handle (36); the protective cover (22) is arc sheet-shaped; a proximal end of the protection cover (22) is connected with a distal end of the protection control member (23), and the distal end of the protection control member (23) is flexible and has a ring-shaped structure; a proximal end of the protection control member (23) is connected with the control handle (36); the protection cover (22) can be controlled to move axially or rotate by operating the control handle (36); a distance between an inner wall of the protective cover (22) and an axis of the tissue removal head (1) is greater than a radius of the tissue removal head (1).

2. The adjustable and bendable tissue removal apparatus according to claim 1, **characterized in that**, the ring-shaped structure is an open ring-shaped structure, and a width of an opening of the open ring-shaped structure is smaller than an outer diameter of the adjustable and bendable unit (11).

3. The adjustable and bendable tissue removal apparatus according to claim 1, **characterized in that**, a distance from an outer side wall of the ring-shaped structure, which is located at a same side where the protective cover (22) is disposed, to an axis of the adjustable and bendable unit (11), is greater than a distance from an opposite outer side wall of the ring-shaped structure to the axis of the adjustable and bendable unit (11).

4. The adjustable and bendable tissue removal apparatus according to claim 3, **characterized in that**, a circumferential wall thickness of the ring-shaped structure is nonuniform, and the protection cover (22) is fixed at a side where the wall is thicker.

5. The adjustable and bendable tissue removal apparatus according to claim 3, **characterized in that**, protrusions are provided on an inner wall of the ring-shaped structure, and the protrusion is dot-shaped or strip-shaped; the protective cover (22) is disposed on a same side where the protrusions are disposed.

6. The adjustable and bendable tissue removal apparatus according to claim 3, **characterized in that**, the protection device further includes a gasket (25) which is disposed between the protection control member (23) and the adjustable and bendable unit (11); the protective cover (22) is fixed on a same side where the gasket (25) is arranged.

7. The adjustable and bendable tissue removal apparatus according to claim 1, **characterized in that**, the protection control member (23) is disposed between the straight adjusting pipe (31) and the adjustable and bendable unit (11), or arranged outside the straight adjusting pipe (31).

8. The adjustable and bendable tissue removal apparatus according to claim 1, **characterized in that**, a longitudinal section of an intermediate portion of the fixed bending part (13) is arc sheet-shaped in structure.

9. The adjustable and bendable tissue removal apparatus according to claim 1, **characterized in that**, the tissue removal head (1) is a grinding head, and a distal end of the rotation shaft (12) is configured to be bended along with bending of the fixed bending part (13).

10. The adjustable and bendable tissue removal apparatus according to claim 1, further including a position limiting device (37); the position limiting device (37) is provided on the protection control member (23) or on the control handle (36); when the protective cover (22) moves towards the tissue removal head (1), the position limiting device (37) is configured to prevent the protective cover (22) from completely passing across the tissue removal head (1).

## Patentansprüche

1. Justierbare und biegbare Gewebeentfernungsvorrichtung, die einen Gewebeentfernungskopf (1), eine justierbare und biegbare Einheit (11), eine Drehwelle (12) umfasst, wobei die justierbare und biegbare Einheit (11) ein gerades Segment (18) an einem proximalen Ende und einen festen Biegeteil (13) an einem distalen Ende aufweist; ein proximales Ende des geraden Segments (18) fest mit einem Reguliergriff (35) verbunden ist; die Drehwelle (12) innerhalb der justierbaren und biegbaren Einheit (11) angeordnet ist; ein distales Ende der Drehwelle mit dem Gewebeentfernungskopf (1) verbunden ist, und ein proximales Ende der Drehwelle mit einem Verbindungselement versehen ist, das mit einer Abtriebswelle des Motors verbunden ist; die justierbare und biegbare Gewebeentnahmevorrichtung ferner eine Schutzvorrichtung aufweist;
wobei die justierbare und biegbare Gewebeentnahmevorrichtung ferner ein gerades Justierrohr (31) aufweist, wobei das gerade Justierrohr (31) außerhalb der justierbaren und biegsamen Einheit (11) ummantelt ist; ein proximales Ende des geraden Justierrohrs (31) mit dem Reguliergriff (35) verbunden ist; das gerade Justierrohr (31) für eine axiale Bewegung durch Betätigen des Reguliergriffs (35) gesteuert werden kann; und der feste Biegeteil (13) aus elastischem Material gefertigt ist;
wobei die Schutzvorrichtung eine Schutzabdeckung (22), ein Schutzsteuerelement (23) und einen Steuergriff (36) umfasst; die Schutzabdeckung (22) bogenblechförmig ist; ein proximales Ende der Schutzabdeckung (22) mit einem distalen Ende des Schutzsteuerelements (23) verbunden ist und das distale Ende des Schutzsteuerelements (23) flexibel ist und eine ringförmige Struktur aufweist; ein proximales Ende des Schutzsteuerelements (23) mit dem Steuergriff (36) verbunden ist; die Schutzabdeckung (22) zum axialen Bewegen oder Drehen durch Betätigen des Steuergriffs (36) gesteuert werden kann; ein Abstand zwischen einer Innenwand der Schutzabdeckung (22) und einer Achse des Gewebeentnahmekopfes (1) größer als ein Radius des Gewebeentnahmekopfes (1) ist.

2. Justierbare und biegbare Gewebeentnahmevorrichtung nach Anspruch 1, **dadurch gekennzeichnet, dass** die ringförmige Struktur eine offene ringförmige Struktur ist und eine Breite einer Öffnung der offenen ringförmigen Struktur kleiner als ein Außendurchmesser der justierbaren und biegbaren Einheit (11) ist.

3. Justierbare und biegbare Gewebeentnahmevorrichtung nach Anspruch 1, **dadurch gekennzeichnet, dass** ein Abstand von einer äußeren Seitenwand der ringförmigen Struktur, die sich auf derselben Seite befindet, auf der die Schutzabdeckung (22) angeordnet ist, zu einer Achse der justierbaren und biegbaren Einheit (11) größer ist als ein Abstand von einer gegenüberliegenden äußeren Seitenwand der ringförmigen Struktur zur Achse der justierbaren und biegbaren Einheit (11) .

4. Justierbare und biegbare Gewebeentnahmevorrichtung nach Anspruch 3, **dadurch gekennzeichnet, dass** eine Umfangswanddicke der ringförmigen Struktur ungleichförmig ist und die Schutzabdeckung (22) an einer Seite befestigt ist, an der die Wand dicker ist.

5. Justierbare und biegbare Gewebeentnahmevorrichtung nach Anspruch 3, **dadurch gekennzeichnet, dass** Vorsprünge an einer Innenwand der ringförmigen Struktur vorgesehen sind und der Vorsprung punkt- oder streifenförmig ist; die Schutzabdeckung (22) auf der gleichen Seite angeordnet ist, auf der die Vorsprünge angeordnet sind.

6. Justierbare und biegbare Gewebeentnahmevorrichtung nach Anspruch 3, **dadurch gekennzeichnet, dass** die Schutzvorrichtung ferner eine Dichtung (25) aufweist, die zwischen dem Schutzsteuerelement (23) und der justierbaren und biegbaren Einheit (11) angeordnet ist; die Schutzabdeckung (22) auf derselben Seite befestigt ist, auf der die Dichtung (25) angeordnet ist.

7. Justierbare und biegbare Gewebeentnahmevorrichtung nach Anspruch 1, **dadurch gekennzeichnet, dass** das Schutzsteuerelement (23) zwischen dem geraden Justierrohr (31) und der justierbaren und biegbaren Einheit (11) oder außerhalb des geraden Justierrohrs (31) angeordnet ist.

8. Justierbare und biegbare Gewebeentnahmevorrichtung nach Anspruch 1, **dadurch gekennzeichnet, dass** eine longitudinale Sektion eines Zwischenabschnitts des festen Biegeteils (13) eine bogenblechförmige Struktur aufweist.

9. Justierbare und biegbare Gewebeentnahmevorrichtung nach Anspruch 1, **dadurch gekennzeichnet, dass** der Gewebeentnahmekopf (1) ein Schleifkopf ist und ein distales Ende der Drehwelle (12) so konfiguriert ist, dass es gemeinsam mit dem Biegen des festen Biegeteils (13) gebogen wird.

10. Justierbare und biegbare Gewebeentnahmevorrichtung nach Anspruch 1, die ferner eine Positionsbegrenzungsvorrichtung (37) beinhaltet; wobei die Positionsbegrenzungsvorrichtung (37) am Schutzsteuerelement (23) oder am Steuergriff (36) vorgesehen ist; die Positionsbegrenzungsvorrichtung (37) so konfiguriert ist, dass sie, wenn sich die Schutzabdeckung (22) zum Gewebeentnahmekopf (1) hin bewegt, verhindert, dass die Schutzabdeckung (22) vollständig über den Gewebeentnahmekopf (1) läuft.

## Revendications

1. Appareil d'ablation tissulaire réglable et souple, comprenant une tête d'ablation tissulaire (1), une unité réglable et souple (11), un arbre de rotation (12), l'unité réglable et souple (11) comportant un segment rectiligne (18) à une extrémité proximale et une partie souple fixe (13) à une extrémité distale ; une extrémité proximale du segment rectiligne (18) est raccordée de manière fixe à une poignée de régulation (35) ; l'arbre de rotation (12) est disposé à l'intérieur de l'unité réglable et souple (11) ; une extrémité distale de l'arbre de rotation est raccordée à la tête d'ablation tissulaire (1), et une extrémité proximale de l'arbre de rotation est dotée d'un élément de raccordement qui raccordé à un arbre de sortie du moteur, l'appareil d'ablation tissulaire réglable et souple comportant en outre un dispositif de protection ;
dans lequel
l'appareil d'ablation tissulaire réglable et souple comprend en outre un tube d'ajustement rectiligne (31), le tube d'ajustement rectiligne (31) étant enveloppé en dehors de l'unité réglable et souple (11) ; une extrémité proximale du tube d'ajustement rectiligne (31) est raccordée à la poignée de régulation (35) ; le tube d'ajustement rectiligne (31) peut être commandé pour se déplacer axialement par actionnement de la poignée de régulation (35) ; et la partie souple fixe (13) est réalisée dans un matériau élastique ;
dans lequel
le dispositif de protection comprend un couvercle de protection (22), un élément de contrôle de protection (23) et une poignée de commande (36) ; le couvercle de protection (22) est en forme de feuille incurvée ; une extrémité proximale du couvercle de protection (22) est raccordée à une extrémité distale de l'élément de contrôle de protection (23), et l'extrémité distale de l'élément de contrôle de protection (23) est souple et a une structure en forme d'anneau ; une extrémité proximale de l'élément de contrôle de protection (23) est raccordée à la poignée de commande (36) ; le couvercle de protection (22) peut être commandé pour se déplacer axialement ou tourner par actionnement de la poignée de commande (36) ; une distance entre une paroi interne du couvercle de protection (22) et un axe de la tête d'ablation tissulaire (1) est supérieure à un rayon de la tête d'ablation tissulaire (1).

2. Appareil d'ablation tissulaire réglable et souple selon la revendication 1, **caractérisé en ce que**, la structure en forme d'anneau est une structure en forme d'anneau ouverte, et une largeur d'une ouverture de la structure en forme d'anneau ouverte est inférieure à un diamètre extérieur de l'unité réglable et souple (11).

3. Appareil d'ablation tissulaire réglable et souple selon la revendication 1, **caractérisé en ce qu'**une distance depuis une paroi latérale externe de la structure en forme d'anneau, qui est située du même côté que le couvercle de protection (22), jusqu'à un axe de l'unité réglable et souple (11), est supérieure à une distance depuis une paroi latérale externe opposée de la structure en forme d'anneau jusqu'à l'axe de l'unité réglable et souple (11).

4. Appareil d'ablation tissulaire réglable et souple selon la revendication 3, **caractérisé en ce qu'**une épaisseur de paroi circonférentielle de la structure en forme d'anneau est non en uniforme, et le couvercle de protection (22) est fixé du côté où la paroi est la plus épaisse.

5. Appareil d'ablation tissulaire réglable et souple selon la revendication 3, **caractérisé en ce que** des protubérances sont ménagées sur une paroi interne de la structure en forme d'anneau, et la protubérance est en forme de point ou en forme de bande ; le couvercle de protection (22) est disposé du même côté que les protubérances.

6. Appareil d'ablation tissulaire réglable et souple selon la revendication 3, **caractérisé en ce que** le dispositif de protection comporte en outre un joint (25) qui est disposé entre l'élément de contrôle de protection (23) et l'unité réglable et souple (11) ; le couvercle de protection (22) étant fixé sur le même côté que le joint (25).

7. Appareil d'ablation tissulaire réglable et souple selon la revendication 1, **caractérisé en ce que** l'élément de contrôle de protection (23) est disposé entre le tube d'ajustement rectiligne (31) et l'unité ajustable et souple (11), ou disposé en dehors du tube d'ajustement rectiligne (31).

8. Appareil d'ablation tissulaire réglable et souple selon la revendication 1, **caractérisé en ce qu'**une section longitudinale d'une partie intermédiaire de la partie souple fixe (13) est une structure en forme de feuille incurvée.

9. Appareil d'ablation tissulaire réglable et souple selon la revendication 1, **caractérisé en ce que** la tête d'ablation tissulaire (1) est une tête de meulage, et une extrémité distale de l'arbre de rotation (12) est configurée pour être fléchie lors du fléchissement de la partie souple fixe (13).

10. Appareil d'ablation tissulaire ajustable et souple selon la revendication 1, comportant en outre un dispositif de limitation de position (37) ; le dispositif de limitation de position (37) étant fourni sur l'élément de contrôle de protection (23) ou sur la poignée de commande (36) ; quand le couvercle de protection (22) se déplace vers la tête d'ablation tissulaire (1), le dispositif de limitation de position (37) est configuré pour empêcher le couvercle de protection (22) de passer complètement en travers de la tête d'ablation tissulaire (1).
